# EUROPÄISCHE PATENTSCHRIFT

(11) **EP 3 697 753 B1**
(45) Veröffentlichungstag und Bekanntmachung des Hinweises auf die Patenterteilung: **01.10.2025**
(21) Anmeldenummer: 18779712.1
(22) Anmeldetag: 08.10.2018
(51) Int. Cl.: C07C 209/36, C07C 211/51

(54) **ERHÖHUNG DER KATALYSATORSELEKTIVITÄT BEI DER KONTINUIERLICHEN HYDRIERUNG VON NITROVERBINDUNGEN DURCH ZUGABE VON AMMONIAK**
INCREASE OF THE CATALYST SELECTIVITY IN THE CONTINUOUS HYDROGENATION OF NITRO COMPOUNDS BY ADDITION OF AMMONIA
AUGMENTATION DE LA SÉLECTIVITÉ DU CATALYSEUR LORS D'HYDROGÉNATION CONTINUE DE COMPOSÉS NITRÉS PAR ADDITION D'AMMONIAC

(30) Priorität: 16.10.2017 EP 17196607
(43) Veröffentlichungstag der Anmeldung: 26.08.2020
(73) Patentinhaber: BASF SE, 67056 Ludwigshafen am Rhein (DE)
(72) Erfinder: WUCHER, Barbara, 67056 Ludwigshafen (DE); HEIDEMANN, Thomas, 67056 Ludwigshafen (DE); FRIKO, Michael, 67056 Ludwigshafen (DE); BECHTOLD, Christian, 67056 Ludwigshafen (DE)
(74) Vertreter: BASF IP Association
(86) Internationale Anmeldenummer: PCT/EP2018/077238
(87) Internationale Veröffentlichungsnummer: WO 2019/076658

(56) Entgegenhaltungen:
- CN-A- 103 387 498
- CN-A- 104 710 316
- DE-A1- 10 105 277
- DE-A1- 102005 041 532
- DE-A1- 2 461 615
- DE-A1- 3 928 329

## Beschreibung

Die vorliegende Erfindung betrifft ein Verfahren zur kontinuierlichen Hydrierung einer Nitroverbindung zu dem entsprechenden Amin in einem die Nitroverbindung enthaltenden flüssigen Reaktionsgemisch in einem Reaktionsraum in Gegenwart eines Trägerkatalysators, der als aktive Komponente mindestens ein Element aus den Gruppen 7 bis 12 des Periodensystems der Elemente enthält, dadurch gekennzeichnet, dass während der Hydrierung Ammoniak in den Reaktionsraum zugegeben wird.

Verfahren zur kontinuierlichen Hydrierung von Nitroverbindungen zu den entsprechenden Aminen sind an sich bekannt.

Eine häufig beschriebene Schwierigkeit bei Verfahren zur Hydrierung von Nitroverbindungen zu den entsprechenden Aminen ist die Freisetzung großer Mengen an Reaktionswärme und damit verbundene potenziell hohe Reaktionstemperaturen.

So beschreibt DE 10 2008 063308 B4 ein Verfahren zur Herstellung von Toluylendiamin durch Hydrierung von Dinitrotoluol, bei dem die Reaktionswärme zur Gewinnung von Dampf genutzt wird, welcher in ein Dampfnetz einer industriellen Anlage eingespeist und weiter genutzt werden kann. Zur Dampferzeugung sind zwingend Reaktionstemperaturen größer 100° C erforderlich. Die in DE 10 2008 063308 B4 offenbarte Hydrierung wird bei einer Temperatur größer oder gleich 180° C durchgeführt.

Ein häufig beschriebenes Problem hoher Reaktionstemperaturen während einer Hydrierung sind jedoch unerwünschte Nebenreaktionen, welche beispielsweise auch durch örtliche Überhitzungen innerhalb des Reaktors ablaufen.

WO 2014/108351 A1 beschreibt eine Vorrichtung des Typs Loop-Venturi-Reaktor für die kontinuierliche Umsetzung von Flüssigkeiten mit Gasen, insbesondere für Hydrierungen, Oxidationen oder Acetylierungen, z.B. für die Herstellung von Toluylendiamin durch Hydrierung von Dinitrotoluol. Lokale Temperaturspitzen bei großer freiwerdender Reaktionswärme werden durch Modifizierung der Anordnung der Wärmeübertragerrohre des Reaktors vermieden. Als Katalysatoren werden beispielsweise aktivierte Nickelkatalysatoren gemäß WO 2008/145179 A1 verwendet, welche aus einer dotierten Ni/Al-Legierung bestehen und nicht geträgert sind. Ein Problem solcher Ni/Al-Legierungen ist die Entstehung von Nickelaluminaten, wie Takovit oder Takovit ähnlichen Verbindungen. Nickelaluminate entstehen bei der Hydrierung von Nitroaromaten, wenn der Ni/Al-Katalysator mehr als 5,5 Gew.-% Al enthält. Sie bilden Feststoffe, welche sich an den Wänden des Reaktors und den peripheren Apparaturen, wie Pumpen, festsetzen. Dies kann zur Verringerung der Effizienz der Wärmeübertragung des Systems und sogar zu Verstopfungen des Systems führen. Zur Dotierung der Ni/Al-Legierung des aktivierten Nickelkatalysators aus WO 2008/145179 A1 werden eines oder mehrere Metalle ausgewählt aus der Gruppe bestehend aus Mg, Ce, Ti, V, Nb, Cr, W, Mn, Re, Fe, Ru, Co, Rh, Ir, Pt, Cu, Ag, Au und Bi verwendet, um die Bildung von Nickelaluminaten, wie Takovit oder Takovit ähnlichen Verbindungen, zu reduzieren oder ganz zu vermeiden.

EP 1678118 B1 betrifft ein Verfahren zur Herstellung von Aminen durch katalytische Hydrierung der entsprechenden Nitroverbindungen. Um Nebenreaktionen, welche zur Bildung von hochmolekularen Nebenprodukten oder zur Bildung von Leichtsiedern führen, zurückzudrängen, wird die Selektivität des Verfahrens mithilfe eines Katalysators bestehend aus Platin und Nickel verbessert.

DE 10 2005 041532 A1 beschreibt ebenfalls ein Verfahren zur Herstellung von Aminen durch katalytische Hydrierung der entsprechenden Nitroverbindungen mit verminderten Nebenreaktionen und verbesserter Selektivität des Verfahrens unter Verwendung eines Katalysators bestehend aus Platin, Nickel und einem zusätzlichen Metall.

Ein weiteres Problem bei den bekannten Verfahren besteht darin, dass sich nach längerer Laufzeit des Reaktionsprozesses aufgrund der Alterung des Katalysators die Produktausbeute verringert. Beispielsweise können Verunreinigungen im zugeführten Edukt oder die Produkte unerwünschter Nebenreaktionen zur Alterung des Katalysators beitragen. Die Alterung des Katalysators kann zudem beschleunigt werden durch eine Unterbrechung in der Zufuhr des Edukts, beispielsweise während einer Betriebspause.

Bei Verwendung eines Reaktors mit Stoffrückführung, einem sogenannten Schlaufenreaktor, kann es Bereiche geben, in denen eine vollständige Umwandlung des Edukts stattfindet. In diesen Bereichen kann die Alterung des Katalysators beschleunigt sein, da durch die vollständige Umwandlung des Edukts die Bildung hochsiedender Komponenten, wie verschieden amino- und methyl-substituierte Diphenylamine, Hydrazodiphenyle und Phenazine, vermehrt stattfindet.

Weiterhin führt eine Unterbrechung der Zufuhr von Nitroverbindungen bei gleichzeitiger Aufrechterhaltung der Reaktionsbedingungen (wie Temperatur, Druck, Wasserstoffstrom und Strömung im Schlaufenreaktor (Zirkulationsstrom)) sowie aller anderen Bedingungen zur Bildung hochsiedender Komponenten, wie verschiedene amino- und methyl-substituierte Diphenylamine, Hydrazodiphenyle und Phenazine, welche den Katalysator deaktivieren können. Wird das Verfahren durch erneute Zufuhr von Nitroverbindungen fortgeführt, ist die Ausbeute an Aminen deutlich geringer und erholt sich im Allgemeinen nur langsam und unvollständig.

DE10105277A1 beschreibt ein Verfahren zur Herstellung von Aminen durch Hydrierung der entsprechenden Nitroverbindungen, bei dem der im Reaktor vorliegende Wasserstoff Anteile mindestens eines bei der Hydrierreaktion inerten Gases enthält.

Aufgabe der vorliegenden Erfindung ist es, in einem Verfahren zur kontinuierlichen Hydrierung von Nitroverbindungen zu den entsprechenden Aminen die Aktivität des Katalysators längerfristig zu erhalten, ohne die Zufuhrrate von Edukten oder die Temperatur reduzieren zu müssen. Darüber hinaus sollte die Selektivität des Katalysators erhöht werden. Weiterhin ist es eine Aufgabe der vorliegenden Erfindung, die Bildung hochsiedender Komponenten, die zur Verunreinigung des Katalysators sowie zu dessen Deaktivierung führen, zu verringern.

Die Aufgabe wird gelöst durch ein Verfahren zur kontinuierlichen Hydrierung eines Nitroaromaten zu dem entsprechenden Amin in einem die Nitroverbindung enthaltenden flüssigen Reaktionsgemisch in einem Reaktionsraum in Gegenwart eines Trägerkatalysators, der als aktive Komponente mindestens ein Element aus den Gruppen 7 bis 12 des Periodensystems der Elemente enthält, dadurch gekennzeichnet, dass während der Hydrierung Ammoniak in den Reaktionsraum zugegeben wird, wobei die zugegebene Menge an Ammoniak mindestens 200 mmol und höchstens 3000 mmol pro kg zugegebene Nitroverbindung beträgt und es sich bei der zu hydrierenden Nitroverbindung um Dinitrotoluol handelt.

Es wurde überraschend gefunden, dass die Zugabe von Ammoniak während der Hydrierung die Produktausbeute an Aminen signifikant erhöht. Gleichzeitig wird die Bildung hochsiedender Komponenten während des Verfahrens verringert und die Lebenszeit des Katalysators gesteigert. Gleichzeitig lässt sich das Ammoniak aus dem Reaktionsprodukt leicht entfernen, beispielsweise durch destillative Methoden.

Überraschenderweise verringert sich auch die Bildung hochsiedender Komponenten, wenn sich vor einer Unterbrechung der Zufuhr von Nitroverbindungen Ammoniak in dem Reaktor befindet. Die Reaktionsausbeute an Aminen erreicht unmittelbar nach Weiterführung der Reaktion durch Zufuhr von Nitroverbindungen wieder ihr ursprüngliches Niveau.

Das erfindungsgemäße Verfahren zur kontinuierlichen Hydrierung einer Nitroverbindung zu dem entsprechenden Amin in einem die Nitroverbindung enthaltenden flüssigen Reaktionsgemisch in einem Reaktionsraum in Gegenwart eines Trägerkatalysators, der als aktive Komponente mindestens ein Element aus den Gruppen 7 bis 12 des Periodensystems der Elemente enthält, ist dadurch gekennzeichnet, dass während der Hydrierung Ammoniak in den Reaktionsraum zugegeben wird.

Während der Hydrierung Ammoniak in den Reaktionsraum zugeben bedeutet, dass die Zugabe des Ammoniaks kontinuierlich mit den anderen Edukten erfolgt, während die Reaktion nämlich die Hydrierung stattfindet und beinhaltet nicht eine Vorlage der gewünschten Ammoniakmenge vor dem eigentlichen Reaktionsstart, dabei ist unter Reaktionsraum ein Raum zu verstehen, innerhalb dessen die kontinuierliche Hydrierung der Nitroverbindung erfolgt. Im Allgemeinen enthält die aktive Komponente des Trägerkatalysators mindestens ein Element der Gruppe bestehend aus Nickel, Platin, Palladium, Eisen und Kobalt.

In einer ersten bevorzugten Ausführungsform enthält die aktive Komponente des Trägerkatalysators Nickel in Form von Nickelkristalliten mit einer bimodalen Nickelkristallitgrößenverteilung und weist einen Nickel-Gehalt von 60 bis 80 Gew.-% bezogen auf die Gesamtmasse des Katalysators sowie einen Reduktionsgrad von mindestens 70% auf.

In einer zweiten bevorzugten Ausführungsform enthält die aktive Komponente des Trägerkatalysators ein Gemisch aus Nickel und Platin sowie gegebenenfalls mindestens einem zusätzlichen Metall. Vorzugsweise enthält der Hydrierkatalysator dieser zweiten bevorzugten Ausführungsform 1 bis 5 Gew.-% Platin, 0,3 bis 1,5 Gew.-% Nickel, 0,05 bis 1,5 Gew.-% des mindestens einen zusätzlichen Metalls, sowie 94,65 bis 97,45 Gew.-% Trägermaterial bezogen auf das Gesamtgewicht des Katalysators. Vorzugsweise ist das mindestens eine zusätzliche Metall Chrom.

### Nitroverbindungen

Geeignete Nitroverbindungen sind Dinitrotoluol .

In einer ganz besonders bevorzugten Ausführungsform wird 2,4-Dinitrotoluol oder 2,6-Dinitrotoluol eingesetzt. Auch technische Gemische enthaltend 2,4-Dinitrotoluol und 2,6-Dinitrotoluol sind geeignet, wobei diese Gemische vorzugsweise bis zu 35 Gew.-% an 2,6-Dinitrotoluol mit Anteilen von vorzugsweise 1 bis 5 Gew.-%, bevorzugt 1 bis 4 Gew.-% an vicinalem Dinitrotoluol und vorzugsweise 0,5 bis 1,5 Gew.-% an 2,5- und 3,5-Dinitrotoluol, bezogen auf das Gesamtgemisch, aufweisen.

Die genannten Nitroaromaten sind kommerziell erhältlich.

Weiterhin können die verwendeten Nitroaromaten durch chemische Synthese gewonnen werden, wie beispielsweise Dinitrotoluole durch die Nitrierung von Toluol erhalten werden können. Das dabei entstehende Reaktionsprodukt enthält zumeist neben der gewünschten Nitroverbindung zahlreiche Verunreinigungen. Beispielsweise kann Nitriersäure umfassend Salpetersäure, Schwefelsäure und Stickstoffoxide in diesem Reaktionsprodukt enthalten sein. Auch Abbauprodukte, wie beispielsweise Distickstoffmonoxid, Cyanwasserstoffsäure, Kohlenstoffmonoxid oder Gemische daraus, können als Verunreinigungen enthalten sein. Ebenso können Oxidationsprodukte, beispielsweise aus unerwünschten Nebenreaktionen der Nitroaromaten, enthalten sein, wie z.B. aromatische Carbonsäuren, wie Nitrobenzoesäuren, beispielsweise Mononitrobenzoesäure, Dinitrobenzoesäure oder deren Abbauprodukte sowie Gemische daraus.

Weitere Verunreinigungen können in Form von Hochsiedern, wie Nitrokresol, wie beispielsweise Mononitrokresol, Dinitrokresol oder Trinitrokresol, und Nitrophenol, wie beispielsweise Dinitrophenol oder Trinitrophenol, vorliegen.

Im Allgemeinen ist daher eine Aufreinigung der so erhaltenen Nitroverbindungen notwendig, damit sie als Edukte für nachfolgende Verfahren, wie beispielsweise die Hydrierung zu den entsprechenden Aminen, geeignet sind. Verfahren zur Synthese von Nitroverbindungen sowie deren weitere Aufreinigung sind dem Fachmann allgemein bekannt oder für Dinitrotoluol in US 2014/0039227 A1 beschrieben.

Die Aufreinigung erfolgt im Allgemeinen in einem mehrstufigen Waschverfahren umfassend mindestens drei Waschschritte: einen Waschschritt zur Entfernung der Säuren, einen Waschschritt in Gegenwart einer Base zum Entfernen schwacher Säuren sowie einen neutralen Waschschritt zum Entfernen verbliebener alkalischer Substanzen.

Die resultierenden Waschlösungen können beispielsweise Nitrokresole oder Nitroaromaten enthalten, welche beispielsweise durch Fällung mithilfe von Ansäuerung, durch Behandlung mit Aktivkohle, durch stark basische Austauschersäulen, durch Extraktion mithilfe von Toluol oder dem zu nitrierenden Aromaten, durch Oxidation mithilfe von Salpetersäure und anschließender thermischer Zersetzung, durch Zersetzung mithilfe von Wasserstoffperoxid oder Ozon, oder durch Thermolyse von den Nitroaromaten abgetrennt werden können.

Nach der Abtrennung von Nitriersäure sowie potentiell vorhandener Nitrokresole und/oder Nitrobenzoesäuren kann die Aufreinigung der Nitroaromaten wie in US 2014/0039227 A1 für Dinitrotoluol beschrieben auch in zwei Waschschritten erfolgen. In einem ersten Waschschritt umfassend mindestens einen Extraktionsschritt kann die Rohmischung mit einer ersten Waschsäure enthaltend Salpetersäure, Stickstoffoxide und Schwefelsäure gewaschen werden. Im Allgemeinen hat die bei der Extraktion abgeführte Waschlösung einen Gesamtgehalt an Säure von 20 bis 40 Gew.-%, bezogen auf das Gesamtgewicht dieser Waschlösung, und enthält beispielsweise Salpetersäure, Stickstoffoxide, wie salpetrige Säure, und Schwefelsäure.

Die so verbliebene Mischung, welche die gewünschte Nitroverbindung enthält, wird beispielsweise in einem zweiten Waschschritt mit einer zweiten Waschsäure, in mindestens einem weiteren Extraktionsschritt behandelt. Bei diesem Extraktionsschritt hat die abgeführte Waschlösung im Allgemeinen einen pH-Wert von ≤ 4. Üblicherweise wird eine Mischung enthaltend die gewünschte Nitroverbindung erhalten, die grundsätzlich frei von Salpetersäure, Schwefelsäure und Stickstoffoxiden sein kann. Die auf diese Weise erhaltene aufgereinigte Nitroverbindung kann im Allgemeinen zur Hydrierung zu dem entsprechenden Amin verwendet werden.

Die aus dem oben genannten Aufreinigungsverfahren erhaltene oder die zur Hydrierung verwendete Nitroverbindung hat im Allgemeinen einen Restsäuregehalt von ≤ 300 ppm, wie beispielsweise Schwefelsäure, und einen pH-Wert von 2 bis 4. Im Allgemeinen enthält die Nitroverbindung,Dinitrotoluol, ≤ 800 ppm Nitrokresole oder Nitrophenole oder Gemische daraus, ≤ 600 pm Nitrobenzoesäure, wie z.B. Mononitrobenzoesäureoder Dinitrobenzoesäure oder Gemische daraus, sowie einen Restgehalt von ≤ 300 ppm, bevorzugt ≤ 200 ppm und besonders bevorzugt ≤ 100 ppm Salpetersäure oder salpetrige Säure oder Gemische daraus, ≤ 50 ppm, bevorzugt ≤ 10 ppm und besonders bevorzugt ≤ 1 ppm Cyanwasserstoffsäure, ≤ 200 ppm, bevorzugt ≤ 50 ppm und besonders bevorzugt ≤ 25 ppm Distickstoffoxid, ≤ 400 ppm, bevorzugt ≤ 200 ppm und besonders bevorzugt ≤ 50 ppm Stickstoffmonoxid, sowie ≤ 3 ppm Sulfat.

In einer bevorzugten Ausführungsform enthält das Reaktionsgemisch neben der zu hydrierenden Nitroverbindung mindestens einen Hochsieder aus der Gruppe bestehend aus Nitrokresolen und Nitrophenolen. In einer besonders bevorzugten Ausführungsform enthält das Reaktionsgemisch neben der zu hydrierenden Nitroverbindung mindestens einen Hochsieder aus der Gruppe bestehend aus Dinitrokresolen, Trinitrokresolen und Nitrophenolen.

In einer anderen bevorzugten Ausführungsform enthält das Reaktionsgemisch neben der zu hydrierende Nitroverbindung keine Hochsieder aus der Gruppe bestehend aus Nitrokresolen und Nitrophenolen. In einer anderen besonders bevorzugten Ausführungsform enthält das Reaktionsgemisch neben der zu hydrierende Nitroverbindung keine Hochsieder aus der Gruppe bestehend aus Dinitrokresolen, Trinitrokresolen und Nitrophenolen
In einer weiteren bevorzugten Ausführungsform enthält das Reaktionsgemisch neben der zu hydrierenden Nitroverbindung mindestens eine Verbindung aus der Gruppe bestehend aus Salpetersäure, Schwefelsäure, Stickstoffoxide, Distickstoffmonoxid, Cyanwasserstoffsäure, Kohlenstoffmonoxid und Nitrobenzoesäure oder deren Abbauprodukte.

Für das erfindungsgemäße Hydrierverfahren kann die Nitroverbindung in reiner Form, als Mischung mit dem entsprechenden Mono-, Di- oder Polyamin, als Mischung mit dem entsprechenden Mono-, Di- oder Polyamin und Wasser, als Mischung mit dem entsprechenden Mono-, Di- oder Polyamin, Wasser und einem alkoholischen Lösungsmittel oder als Mischung mit dem entsprechenden Di- oder Polyamin, Wasser, einem alkoholischen Lösungsmittel und einem katalysatorreaktivierenden Zusatz eingesetzt werden, wobei jeweils auch Gemische aus zwei oder mehr der oben genannten Nitroverbindungen, der entsprechenden Aminverbindungen, dem alkoholischen Lösungsmittel und dem katalysatorreaktivierenden Zusatz eingesetzt werden können.

Als katalysatorreaktivierende Zusätze werden vorzugsweise aprotische Lösungsmittel, insbesondere, Dimethylformamid (DMF), Dioxan oder Tetrahydrofuran (THF) oder ein Gemisch aus zwei oder mehr davon eingesetzt.

Geeignete alkoholische Lösungsmittel sind im Allgemeinen niedere aliphatische Alkohole mit 1 bis 6 C-Atomen. Bevorzugt werden Methanol, Ethanol oder Propanol einzeln oder in einem Gemisch aus zwei oder mehr davon verwendet. Besonders bevorzugt wird Ethanol verwendet.

Sofern ein oben beschriebenes Gemisch eingesetzt wird, liegt das Gewichtsverhältnis von Aminverbindung zu Wasser vorzugsweise im Bereich von 10:1 bis 1:10, bevorzugt im Bereich von 8:1 bis 1:5 und besonders bevorzugt im Bereich von 4:1 bis 1:1 und das Gewichtsverhältnis des Amin/Wasser-Gemischs zu mindestens einem alkoholischen Lösungsmittel vorzugsweise bei 1000:1 bis 1:1, bevorzugt bei 500:1 bis 2,5:1 und besonders bevorzugt bei 50:1 bis 5:1.

Die Menge der eingesetzten alkoholischen Lösungsmittel und der katalysatorreaktivierenden Zusätze ist im Rahmen des erfindungsgemäßen Verfahrens nicht in besonderer Weise beschränkt und kann je nach Bedarf frei gewählt werden.

Das erfindungsgemäße Verfahren zur Hydrierung von Nitroverbindungen zu den entsprechenden Aminen kann zudem in Abwesenheit von Lösungsmitteln durchgeführt werden. Bei dieser Verfahrensweise vereinfacht sich die Aufarbeitung des Reaktionsgemisches nach der Hydrierung, außerdem werden Nebenreaktionen mit dem Lösungsmittel völlig unterbunden. Die aufgereinigte Nitroverbindung kann im Allgemeinen zur Hydrierung zu den entsprechenden Aminen verwendet werden. Die verbliebenen Verunreinigungen sowie die während der Hydrierung entstehenden Nebenprodukte, wie beispielsweise Hochsieder, können jedoch zur Alterung des Katalysators beitragen.

### Ammoniak

Die zugegebene Menge an Ammoniak beträgt mindestens 200 mmol pro kg zugegebener Nitroverbindung, insbesondere mindestens 400 mmol, besonders bevorzugt mindestens 1000 mmol, ganz besonders bevorzugt mindestens 1200 mmol.

Die zugegebene Menge an Ammoniak beträgt höchstens 3000 mmol pro kg zugegebener Nitroverbindung, insbesondere höchstens 2500 mmol, besonders bevorzugt höchstens 2000 mmol, ganz besonders bevorzugt höchstens 1800 mmol.

Ammoniak wird in einer Menge von 200 bis 3000 mmol pro kg zugegebener Nitroverbindung, insbesondere von 400 bis 2500 mmol, besonders bevorzugt von 1000 bis 2000 mmol, ganz besonders bevorzugt von 1200 bis 1800 mmol zugegeben.

Das Ammoniak kann in einer ersten Ausführungsform als flüssiges Ammoniak in oben genannten bevorzugten Mengen zugegeben werden.

Das Ammoniak kann in einer zweiten Ausführungsform als wässrige Lösung in oben genannten bevorzugten Mengen zugegeben werden. Dabei beträgt die Menge des Ammoniaks bezogen auf das Gesamtgewicht der wässrigen Ammoniaklösung vorzugsweise mindestens 1 Gew.-%, insbesondere mindestens 5 Gew.-%, besonders bevorzugt mindestens 10 Gew.- %, ganz besonders bevorzugt mindestens 20 Gew.-%.

Die bevorzugte Art der Zugabe des Ammoniak wird weiter unten im Rahmen der Durchführung des Verfahrens näher erläutert.

### Katalysator

Unter Trägerkatalysator ist ein Katalysator zu verstehen, bei dem sich eine aktive Komponente auf einer inaktiven Komponente befindet (dem Trägermaterial), insbesondere auf dem Trägermaterial verteilt ist. Die aktive Komponente ist die katalytisch aktive Komponente.

Geeignete Trägerkatalysatoren für ein Verfahren zur kontinuierlichen Hydrierung von Nitroverbindungen zu den entsprechenden Aminen in der Flüssigphase sind dem Fachmann allgemein bekannt oder beispielsweise in EP 1 678 118 B1, DE 10 2005 041 532 A1, WO 2008/138784 A1 und US 6,140,539 beschrieben.

Geeignete aktive Komponenten für einen Katalysator zur Hydrierung von Nitroaromaten zu den entsprechenden Aminen sind dem Fachmann allgemein bekannt oder beispielsweise in EP 1 678 118 B1, DE 10 2005 041 532 A1, WO 2008/138784 A1, EP 1161297 A1, EP 1165231 A1 und US 6,140,539 beschrieben.

Im Allgemeinen enthält der Trägerkatalysator als aktive Komponente mindestens ein Element aus den Gruppen 7 bis 12 des Periodensystems der Elemente. Als aktive Komponente des Trägerkatalysators geeignete Elemente aus den Gruppen 7 bis 12 des Periodensystems der Elemente sind beispielsweise Eisen, Kobalt, Nickel, Ruthenium, Rhodium, Palladium, Silber, Osmium, Iridium, Platin, Gold, Kupfer, Rhenium, Zink und/oder Mangan.

Bevorzugt enthält die aktive Komponente des Trägerkatalysators mindestens ein Element aus den Gruppen 7 bis 12 des Periodensystems der Elemente, besonders bevorzugt mindestens ein Element aus der Gruppe bestehend aus Nickel, Platin, Palladium, Eisen und Kobalt, ganz besonders bevorzugt mindestens ein Element aus der Gruppe bestehend aus Nickel, Platin Palladium und Kobalt und insbesondere bevorzugt mindestens Nickel.

Im Allgemeinen enthält der Katalysator 0 bis 10 Gew.-%, bevorzugt 1 bis 5 Gew.-% Edelmetall bezogen auf das Gesamtgewicht des Katalysators.

Optional kann die aktive Komponente des Katalysators neben dem mindestens einen Element aus den Gruppen 7 bis 12 des Periodensystems der Elemente zusätzlich Chrom enthalten.

Um Nebenreaktionen zu unterdrücken, ist es bevorzugt, das Verfahren so zu führen, dass der Katalysator an seiner Belastungsgrenze gefahren wird. Dies kann beispielsweise durch die Menge der zudosierten Nitroverbindung, die Menge des Katalysators im Reaktionsgemisch, die Temperatur oder den Druck gesteuert werden. Unter der Belastungsgrenze des Katalysators im Sinne der Erfindung wird die Menge der hydrierbaren Stickstoffatome und Sauerstoffatome enthaltenden Gruppen verstanden, die vom Katalysator bei gegebenen Druck- und Temperaturbedingungen hydriert werden können. Bei den Stickstoffatome und Sauerstoffatome enthaltenden Gruppen kann es sich neben Nitrogruppen auch um Nitrosogruppen und Nitrosamingruppen handeln.

In einer ersten Ausführungsform kann, wie in US 6,140,539 beschrieben, Nickel als aktive Komponente auf einem Träger verwendet werden, wobei der Katalysator stabilisiert ist, und die Nickelkristallite eine bimodale Nickelkristallitgrößenverteilung, einen Nickel-Gehalt von 60 bis 80 Gew.-%, bezogen auf die Gesamtmasse des Katalysators, sowie einen Reduktionsgrad von mindestens 70% aufweisen.

Die Bestimmung des Reduktionsgrades erfolgt im Allgemeinen bei einer einstündigen Nachreduktion des stabilisierten Katalysators bei 100° C.

Im Allgemeinen liegen die beiden Maxima der bimodalen Nickelkristallitgrößenverteilung bei 30 bis 80 Angström und 81 bis 150 Angström. Bevorzugt beträgt der Anteil des Nickels im Bereich des Maximums von 30 bis 80 Angtröm von ≥ 40 bis <100 Gew.-% bezogen auf die Gesamtmasse des Katalysators.

Als Trägermaterial dieser ersten Ausführungsform sind beispielsweise Oxide und Oxidgemische von Zirkonium, Hafnium oder Silizium geeignet. Vorzugsweise enthält der Träger ZrO₂, ZrO₂HfO₂, SiO₂·ZrO₂ oder SiO₂·ZrO₂HfO₂ oder Gemische enthaltend mindestens zwei dieser Substanzen. Besonders bevorzugt besteht der Träger aus diesen Substanzen.

Bevorzugt beträgt der SiO₂-Gehalt 0 bis 20 Gew.-% bezogen auf die Gesamtmasse des Katalysators.

Vorzugweise beträgt der ZrO₂- Gehalt 0 bis 40 Gew.-% bezogen auf die Gesamtmasse des Katalysators.

Bevorzugt beträgt der HfO₂-Gehalt 0 bis 4 Gew.-% bezogen auf die Gesamtmasse des Katalysators.

Verfahren zur Herstellung eines solchen Katalysators sind dem Fachmann allgemein bekannt oder beispielsweise in US 6,140,539 beschrieben.

Vorzugsweise enthält die aktive Komponente des Trägerkatalysators Nickel in Form von Nickelkristalliten mit einer bimodalen Nickelkristallitgrößenverteilung und weist einen Nickel-Gehalt von 60 bis 80 Gew.-% bezogen auf die Gesamtmasse des Katalysators sowie einen Reduktionsgrad von mindestens 70% auf.

In einer zweiten Ausführungsform wird in dem erfindungsgemäßen Verfahren zur kontinuierlichen Hydrierung von Nitroverbindungen zu den entsprechenden Aminen ein Trägerkatalysator verwendet, dessen aktive Komponente ein Gemisch aus Nickel und Platin sowie gegebenenfalls mindestens eine zusätzliches Metall enthält, wie beispielsweise in EP 1678118 B1 oder DE 10 2005 041 532 A1 beschrieben.

Im Allgemeinen werden aktive Komponenten dieses Katalysators in Form von Gemischen auf das Trägermaterial aufgebracht. Geeignete Gemische enthalten Nickel und Platin mit einem Atomverhältnis von Nickel zu Platin von vorzugsweise zwischen 30:70 und 70:30, bevorzugt zwischen 40:60 und 60:40 und besonders bevorzugt zwischen 45:55 und 55:45. Gemische aus Nickel und Platin mit einem anderen Atomverhältnis sind ebenfalls brauchbar, führen jedoch häufig zu geringen Produktausbeuten.

Bevorzugt wird dem Gemisch enthaltend Nickel und Platin noch mindestens ein zusätzliches Metall hinzugefügt. Als zusätzliches Metall geeignete Metalle sind dem Fachmann allgemein bekannt oder beispielsweise in DE 10 2005 041 532 A1 beschrieben. Bevorzugt ist das zusätzliche Metall mindestens ein Metall aus der Gruppe bestehend aus Kupfer, Kobalt, Eisen, Zink, Mangan und Chrom, besonders bevorzugt mindestens ein Metall aus der Gruppe bestehend aus Kupfer, Kobalt, Eisen und Zink.

Die Metallpartikel sind im Allgemeinen polykristallin. Ihre Charakterisierung ist dem Fachmann allgemein bekannt oder beispielsweise in DE 10 2005 041 532 A1 beschrieben.

Die Beschaffenheit sowie Verfahren zur Charakterisierung des Trägerkatalysators, bei dessen aktivem Material es sich um ein Gemisch aus Nickel und Platin sowie gegebenenfalls mindestens einem zusätzlichen Metall handeln kann, sind dem Fachmann allgemein bekannt oder in EP 1678118 B1 oder DE 10 2005 041 532 A1 beschrieben.

Im Allgemeinen enthält der im erfindungsgemäßen Verfahren verwendete Hydrierkatalysator basierend auf Nickel und Platin und mindestens einem zusätzlichen Metall
1 bis 5 Gew.-% Platin,
0,3 bis 1,5 Gew.-% Nickel,
0,05 bis 1,5 Gew.-% des mindestens einen zusätzlichen Metalls, sowie
94,65 bis 97,45 Gew.-% Trägermaterial
bezogen auf das Gesamtgewicht des Katalysators.

Besonders bevorzugt besteht der im erfindungsgemäßen Verfahren verwendete Hydrierkatalysator basierend auf Nickel und Platin und mindestens einem zusätzlichen Metall aus
1 bis 5 Gew.-% Platin,
0,3 bis 1,5 Gew.-% Nickel,
0,05 bis 1,5 Gew.-% des mindestens einen zusätzlichen Metalls, sowie
94,65 bis 97,45 Gew.-% Trägermaterial
bezogen auf das Gesamtgewicht des Katalysators.

Im Allgemeinen beträgt der Gehalt an Nichtedelmetallen 0 bis 1,6 Gew.-%, bevorzugt 0,1 bis 0,9 Gew.-% bezogen auf das Gesamtgewicht des Katalysators.

Die als Träger für die Katalysatoren dieser zweiten Ausführungsform geeigneten Materialen sind dem Fachmann allgemein bekannt oder beispielsweise in EP 1678118 B1 oder DE 10 2005 041 532 A1 beschrieben. Im Allgemeinen werden Aktivkohle, Ruß, Graphit oder Metalloxide, bevorzugt hydrothermal stabile Metalloxide, wie z.B. ZrO₂ und/oder TiO₂, oder Oxide des Aluminiums, wie Al₂O₃, oder Siliciums oder anderer Materialien eingesetzt.

Vorzugsweise wird Graphit als Träger verwendet, wobei in diesem Fall HSAG (High Surface Area Graphite) mit einer Oberfläche von 50 bis 300 m²/g besonders bevorzugt sind.

Besonders bevorzugt ist die Verwendung von Aktivkohle als Träger. Eine ganz besonders bevorzugte Ausführungsform ist die Verwendung von physisch oder chemisch aktivierter Aktivkohle oder Ruße, wie Acetylenruß, als Träger.

Verfahren zur Herstellung des Trägerkatalysators, bei dessen aktivem Material es sich um ein Gemisch aus Nickel und Platin sowie gegebenenfalls mindestens einem zusätzlichen Metall handelt, sind dem Fachmann allgemein bekannt oder in EP 1678118 B1 oder DE 10 2005 041 532 A1 beschrieben.

Der im erfindungsgemäßen Verfahren verwendete Katalysator basierend auf einem Gemisch aus Nickel und Platin und gegebenenfalls mindestens einem zusätzlichen Metall wird vorzugsweise in einer Menge von 0,01 bis 10 Gew.-%, bevorzugt 0,1 bis 5 Gew.-% und besonders bevorzugt 0,2 bis 2 Gew.-% bezogen auf das Gesamtgewicht des Reaktionsgemisches eingesetzt.

Bevorzugt enthält die aktive Komponente des Trägerkatalysators ein Gemisch aus Nickel und Platin sowie gegebenenfalls mindestens einem zusätzlichen Metall.

Bevorzugt ist das zusätzliche Metall mindestens ein Metall aus der Gruppe bestehend aus Kupfer, Kobalt, Eisen, Zink, Mangan und Chrom, besonders bevorzugt mindestens ein Metall aus der Gruppe bestehend aus Kupfer, Kobalt, Eisen und Zink.

Gemäß einer dritten Ausführungsform des erfindungsgemäßen Verfahrens können Katalysatoren eingesetzt werden, die als aktive Komponente ein Gemisch von Nickel, Palladium und einem zusätzlichen Element, ausgewählt aus der Gruppe, bestehend aus Kobalt, Eisen, Vanadium, Mangan, Chrom, Platin, Iridium, Gold, Bismut, Molybdän, Selen, Tellur, Zinn und Antimon auf einem Träger enthalten. Dieses Verfahren kann zur Herstellung von Toluylendiamin durch Hydrierung von Dinitrotoluol werden. Die Durchführung einer Hydrierung von Nitroverbindungen zu den entsprechenden Aminen mithilfe dieses Katalysators ist dem Fachmann allgemein bekannt oder beispielsweise in der WO 2008/138784 A1 beschrieben.

Das zusätzliche Element ist vorzugsweise ausgewählt aus der Gruppe, bestehend aus Kobalt, Eisen, Vanadium, Bismut und Zinn.

Als Träger für den Katalysator dieser dritten Ausführungsform können im Allgemeinen die hierfür üblichen und bekannten Materialien eingesetzt werden. Vorzugsweise werden Aktivkohle, Ruß, Graphit oder Metalloxide, bevorzugt hydrothermal stabile Metalloxide wie z.B. ZrO₂, TiO₂, Al₂O₃ eingesetzt. Bei Graphit sind die HSAG (High Surface Area Graphite) mit einer Oberfläche von 50 bis 300 m²/g bevorzugt. Besonders bevorzugt sind Aktivkohlen, insbesondere physikalisch oder chemisch aktivierte Aktivkohlen, oder Ruße, wie Acetylenruß.

In einer weiteren Ausführungsform des erfindungsgemäßen Verfahrens ist die aktive Komponente des Katalysators kein Raney-Nickel.

Im Allgemeinen wird der im erfindungsgemäßen Verfahren verwendete Katalysator in einer Menge von 0,01 bis 10 Gew.-%, bevorzugt 0,1 bis 5 Gew.-% und besonders bevorzugt 0,2 bis 2 Gew.-% bezogen auf das Gesamtgewicht des Reaktionsgemisches eingesetzt.

Der Katalysator wird üblicherweise in reduziertem und passiviertem Zustand in den Reaktor eingebracht. Unter dem reduzierten und passiviertem Zustand im Sinne der Erfindung wird verstanden, dass der Katalysator nach der Herstellung aktiviert, ist, danach jedoch aus Sicherheitsgründen die aktiven Zentren beispielsweise durch Überleiten von Sauerstoff oder Kohlendioxid passiviert werden. Geeignet sind zudem der Ausbau und die Stabilisierung des Katalysators unter einer inerten Atmosphäre oder in einem nicht leicht-entzündlichen Lösungsmittel, beispielsweise in Wasser oder einem Gemisch aus Toluendiamin und Wasser, oder höheren Alkoholen, wie z.B. Butanol oder Ethylenglykol.

### Durchführung

Die für das erfindungsgemäße Verfahren geeigneten Reaktoren und Reaktorfahrweisen sind dem Fachmann allgemein bekannt oder beispielsweise in DE10 2005 041 532 A1, DE 10 2008 063 308 B4, WO 2000/035852 A1 oder WO 2014/108351 A1 beschrieben.

Die für das erfindungsgemäße Verfahren anzuwendenden Verfahrensparameter, wie Druck und Temperatur, sind dem Fachmann ebenfalls allgemein bekannt oder beispielsweise in DE 10 2005 041 532 A1, DE 10 2008 063 308 B4, WO 2000/035852 A1 oder WO 2014/108351 A1 beschrieben.

Geeignete Reaktoren sind beispielsweise Rührkessel oder Rohrbündelreaktoren oder Schlaufenreaktoren, wie Strahlschlaufenreaktoren, sogenannte Loop-Venturi-Reaktoren, oder Schlaufenreaktoren mit innerer Strömungsumkehr wie in WO 2000/035852 A1, DE10 2005 041 532 A1, DE 10 2008 063 308 B4 oder WO 2014/108351 A1 beschrieben. Bevorzugt wird ein Schlaufenreaktor für das erfindungsgemäße Verfahren verwendet.

Im Allgemeinen werden die Nitroverbindungen mit einer Rate zugegeben, die einerseits der Katalysatoraktivität angepasst ist und andererseits eine ausreichende Vermischung mit der Strömung im Schlaufenreaktor, dem sogenannten Zirkulationsstrom, ergibt. In der Regel wird die Katalysatoraktivität durch Zugabe ausreichender Katalysatormengen so eingestellt, dass sich der Zugabestrom der Nitroverbindungen nach dem Zirkulationsstrom richtet, so dass lokale Überkonzentrationen von Nitroverbindungen (zum Beispiel größer 10.000 ppm) vermieden werden. Im Fall eines Reaktors mit Strömungsumkehr kann beispielsweise durch den Aufprall des eingespeisten Reaktionsgemischs auf den Reaktorboden oder durch Einbauten eine interne Zirkulationsströmung entstehen, die größer als der Zirkulationsstrom ist. Wird die Nitroverbindung in diesem Fall in die interne Zirkulationsströmung gegeben, richtet sich ihr Zugabestrom nach der Größe der internen Zirkulationsströmung, um bei gegebenem Umsatz in der internen Zirkulationsschlaufe lokale Überkonzentrationen zu vermeiden.

In Strömungsrichtung gesehen nach der ersten Zugabestelle für die Nitroverbindung befindet sich in einer bevorzugten Ausführungsform eine zweite Zugabestelle, durch die zusätzliche Komponenten, insbesondere das erfindungsgemäß zugegebene Ammoniak, in das Verfahren eingespeist werden.

Das Ammoniak kann im Allgemeinen an beliebiger Stelle in den Reaktor und somit in den Reaktionsraum eingespeist werden. Vorzugweise wird Ammoniak nicht zusammen mit dem in dem Reaktor zugeführten Nitroverbindungen gegeben, sondern davon getrennt. Dabei erfolgt die Zugabe vorzugsweise in Strömungsrichtung unmittelbar nach der Zugabe der Nitroverbindung. Dies gewährleistet eine gute und schnelle Durchmischung im gesamten Reaktionsbereich.

Die in dem erfindungsgemäßen Verfahren angewandte gewichtsbezogene Raumgeschwindigkeit beträgt vorzugsweise 5 bis 100 kg (Nitroverbindung)/kg (Katalysator)/h, bevorzugt 10 bis 50 kg (Nitroverbindung)/kg (Katalysator)/h und besonders bevorzugt 15 bis 35 kg (Nitroverbindung)/kg (Katalysator)/h.

Geeignete Hydriergasgemische zur kontinuierlichen Hydrierung von Nitroaromaten zu den entsprechenden Aminen sind dem Fachmann allgemein bekannt oder beispielsweise in EP 1678 118 B1 beschrieben.

Im Allgemeinen können als Hydriergasgemische Gase verwendet werden, die freien Wasserstoff enthalten und keine schädlichen Mengen an Katalysatorgifte, wie beispielsweise Kohlenmonoxid, enthalten. Geeignete Hydriergasgemische sind Reformerabgase, oder Gemische aus Wasserstoff mit Stickstoff und/oder Kohlendioxid. Bevorzugt wird Wasserstoff mit geringem Inertgasanteil als Hydriergasgemisch verwendet.

Die Zufuhr der Hydriergasgemische erfolgt im Allgemeinen abhängig von dem Wasserstoffverbrauch durch die Reaktion, indem der in der Reaktorapparatur herrschende Druck vorzugsweise konstant gehalten wird. Durch kontinuierliche Entnahme eines Teils der Gasphase kann eine Aufpegelung von Inertanteilen im Hydriergasgemisch oder von inerten gasförmigen Reaktionsprodukten vermieden werden.

In einer ersten Ausführungsform kann das erfindungsgemäßen Verfahren, wie in WO 2014/108351 A1 oder DE 10 2008 063 308 B4 beschrieben, bei Temperaturen von 80 bis 200° C, bevorzugt von 110 bis 190° C, besonders bevorzugt von 150 bis 190° C durchgeführt werden. Im Allgemeinen wird das Verfahren dieser ersten Ausführungsform bei Drücken von 10 bis 50 bar, bevorzugt von 15 bis 35 bar durchgeführt. Die dabei entstehende Reaktionswärme kann mit Hilfe von Wärmeüberträgern zur Gewinnung von Dampf mit einem Überdruck von mindestens 4 bar genutzt werden. Die für die Durchführung dieses Verfahrens benötigten Parameter sind dem Fachmann allgemein bekannt oder beispielsweise in WO 2014/108351 A1 oder DE 10 2008 063 308 B4 beschrieben.

Vorzugsweise wird das erfindungsgemäße Verfahren zur Hydrierung von Nitroverbindungen zu Aminen, wie die Hydrierung von Dinitrotoluol zu Toluylendiaminderivaten, in einem vertikal ausgedehnten, wie in WO 2014/108351 A1 beschriebenen, Reaktor durchgeführt, welcher mindestens eine Mischkammer enthält, wobei die Mischkammer oder die Mischkammern an ihrem unteren Ende jeweils mit einem Diffusor verbunden sind.

Für dieses Verfahren geeignete Wärmeüberträger und Kühlmedien sind dem Fachmann ebenfalls allgemeinen bekannt oder beispielsweise in WO 2014/108351 A1 oder DE 10 2008 063 308 B4 beschrieben.

In einer zweiten Ausführungsform kann das erfindungsgemäßen Verfahren wie in WO 00/35852 A1 oder DE 10 2005 041 532 A1 beschrieben bei Temperaturen von vorzugsweise 80 bis 250° C, bevorzugt von 100 bis 200° C und besonders bevorzugt von 120 bis 150° C durchgeführt werden. Im Allgemeinen wird das Verfahren in dieser zweiten Ausführungsform bei Drücken von vorzugsweise 5 bis 100 bar, bevorzugt 10 bis 40 bar und besonders bevorzugt von 20 bis 25 bar durchgeführt.

Geeignete Reaktoren sind dem Fachmann allgemein bekannt oder beispielsweise in WO 00/35852 A1 oder DE 10 2005 041 532 A1 beschrieben. Im Allgemeinen können Rührkessel oder Schlaufenreaktoren, wie Strahlschlaufenreaktoren, sogenannte Loop-Venturi-Reaktoren, oder Schlaufenreaktoren mit innerer Strömungsumkehr eingesetzt werden.

Im Allgemeinen können die bei der Hydrierung gebildeten Amine dem Verfahren kontinuierlich oder diskontinuierlich entnommen werden. Bevorzugt werden die bei der Hydrierung gebildeten Amine dem Verfahren kontinuierlich entnommen.

Die Entnahme der bei der Hydrierung gebildeten Amine kann an beliebiger Stelle erfolgen. Bevorzugt erfolgt die Entnahme aus der externen Zirkulationsströmung vor der Einspeisung der Nitroverbindung. Da die Hydrierung der Nitroverbindung unter den genannten Bedingungen in der internen Zirkulationsströmung im Allgemeinen praktisch quantitativ abläuft, enthält die externe Zirkulationsströmung vor der Einspeisung der Nitroverbindung im Wesentlichen das entsprechende, reine Amin, Wasser, gegebenenfalls Lösungsmittel und Katalysator. Das Amin wird im Allgemeinen aus dem abgezogenen Strom abgetrennt und der Reinigung zugeführt. Der Katalysator und gegebenenfalls Wasser können der externen Zirkulationsströmung wieder zugeführt werden.

Vorzugsweise wird der Katalysator in dem Reaktionsmedium suspendiert. Die Abtrennung des Reaktionsprodukts vom Katalysator erfolgt im Allgemeinen mittels Membranfiltration. Die dazu genutzte Membran kann bevorzugt in der äußeren Umlaufströmung oder beispielsweise in den kontinuierlich gerührten Rührkessel eingebaut werden. Alternativ kann der Katalysator auch durch Sedimentation in einem Settler zurückgehalten werden, der in die äußere Umlaufströmung oder beispielsweise in den kontinuierlich gerührten Rührkessel eingebaut ist.

Die Membranfiltration wird vorzugsweise bei einem Druck auf der Suspensionsseite von 5 bis 50 bar, bevorzugt von 20 bis 35 bar, einer Druckdifferenz zwischen der Suspensionsseite und der Permeatseite von 0,3 bar bis 5 bar und einer Strömungsgeschwindigkeit auf der Suspensionsseite von 1 bis 6 m/s durchgeführt. Unter Suspensionsseite im Sinne der Erfindung wird die Seite des Membranfilters verstanden, auf der sich die Katalysator enthaltende Mischung befindet. Unter Permeatseite im Sinne der Erfindung wird die Seite des Membranfilters verstanden, auf der sich die katalysatorfreie Mischung befindet.

Die Membranfiltration kann kontinuierlich oder diskontinuierlich durchgeführt werden.

Bei der kontinuierlichen Durchführung wird im Allgemeinen ständig zumindest ein Teilstrom des Reaktionsgemisches durch einen Membranfilter gefahren. In einer bevorzugten Ausführungsform des erfindungsgemäßen Verfahrens ist es bevorzugt, den Membranfilter in den externen Kreislauf eines Umlaufreaktors anzuordnen.

Bei der diskontinuierlichen Durchführung der Filtration wird das ausgeschleuste Reaktionsgemisch im Allgemeinen durch eine zuschaltbare Reinigungsstufe, bestehend aus mindestens einem Membranfilter und einer eigenen Zirkulationspumpe, geleitet. Bei einer anderen Ausgestaltung der diskontinuierlichen Filtration wird im Anschluss an die Reaktion das Reaktionsgemisch über einen Membranfilter gefahren.

Die für das Verfahren eingesetzten Filtermembranen können beispielsweise aus Keramik (z.B. α -Al_{g2}O₃) oder Edelstahl (z.B. 1.4404) bestehen und haben, abhängig von der Partikelgröße des eingesetzten Katalysators, bevorzugt zahlengewichtete mittlere Porendurchmesser im Bereich von 10 nm bis 20 Mikrometer, besonders bevorzugt im Bereich von 20 nm bis 10 Mikrometer und ganz besonders bevorzugt von 50 nm bis 1 Mikrometer.

Geeignete Ausführungsformen einer Membranfiltration, insbesondere Querstromfiltration, sind dem Fachmann bekannt und werden beispielsweise in der WO2010/125025 oder WO 2003/066571 beschrieben.

Nach der Abtrennung des Katalysators werden die gebildeten Amine im Allgemeinen weiter aufgereinigt. Verfahren zur Aufreinigung der erfindungsgemäß hergestellten Amine sind dem Fachmann allgemein bekannt oder beispielsweise in WO 2000/035852 A1 beschrieben. Geeignete Verfahren zur Aufreinigung der gebildeten Amine, sind z.B. Destillation oder Extraktion.

### Beispiele

Das folgende Experiment wurde in einer Miniplant-Versuchsanlage durchgeführt. Diese bestand aus einem Schlaufenreaktoraufbau, der in einem Teil (5,6 L) über eine interne Zirkulationsströmung verfügt, die von einem Treibstrahl (Zirkulationsstrom bestehend aus Produkt und Katalysator) angetrieben wird, und in einem anderen Teil als Rohrreaktor ausgeführt ist (4,4 L). Der gesamte Aufbau wurde mit Thermoöl thermostatisiert, um entstehende Wärme abzuführen. Nahe dem Treibstrahl wurde DNT eingemischt und Wasserstoff wurde druckgeregelt in den Gasraum oberhalb der internen Zirkulationsströmung dosiert. Gebildetes Produkt wurde über eine den Katalysator zurückhaltende Membran entnommen, so dass der Flüssigstand im Reaktorteil mit der internen Zirkulationsströmung konstant blieb. Eine feste Gasmenge wurde oberhalb des Gasraums abgeführt, so dass keine unbegrenzte Akkumulation von gasförmigen Produkten oder Verunreinigungen erfolgte.

Der Reaktor wurde mit 112g (Trockenmenge) 3%Pt-1%Ni/C-Katalysator suspendiert in Wasser beladen und bei 185° C, 25 bar Überdruck, Zirkulationsstrom von 500kg/h und einer DNT-Dosierrate von 2kg/h betrieben. Daraus resultierte eine WHSV von 17,9 kg(DNT)/kg(cat)/h.

Die Zugabe von Ammoniak erfolgte durch Zuführung eines kontinuierlichen Volumenstroms einer wässrigen Lösung in den Reaktor. Die Einleitung erfolgte durch ein Rohr, das am Reaktorkopf durchgeführt wird. Die Lösung tropfte dabei in die interne Zirkulationsströmung des Reaktors am Rand des Einsteckrohres, d.h. entfernt von der DNT-Zugabestelle. Durch Variation des Volumenstroms und der Konzentration der Ammoniakwasserlösung wurden die in Tabelle 1 beschriebenen Ammoniak-Konzentrationen eingestellt. Nach Einstellung eines stationären Zustandes wurden mehrere Proben gezogen und per Gaschromatographie analysiert.

Nach einer Einlaufperiode von 250h und einer ausgeprägten Katalysatoralterung wurde Ammoniak hinzugegeben und die TDA-Ausbeute stieg graduell gemäß Tabelle 1 an. **Die** ersten drei Einträge der Tabelle 1 zeigen Vergleichsbeispiele.

**Tabelle 1**

| **mmol(NH3)/kg(DNT)** | **Konzentration zudosiertes NH₃ in Wasser** | **Selektivität TDA** |
|---|---|---|
| 0 | | 97,8 |
| 13 | 2 Gew.-% | 97,8 |
| 199 | 10 Gew.-% | 98,2 |
| 397 | 10 Gew.-% | 98,7 |
| 993 | 25 Gew.-% | 98,9 |
| 1322 | 25 Gew.-% | 99,1 |
| 1656 | 25 Gew.-% | 99,2 |
| 1918 | 25 Gew.-% | 99,0 |

## Patentansprüche

1. Verfahren zur kontinuierlichen Hydrierung eines Nitroaromaten zu dem entsprechenden Amin in einem die Nitroverbindung enthaltenden flüssigen Reaktionsgemisch in einem Reaktionsraum in Gegenwart eines Trägerkatalysators, der als aktive Komponente mindestens ein Element aus den Gruppen 7 bis 12 des Periodensystems der Elemente enthält, **dadurch gekennzeichnet, dass** während der Hydrierung Ammoniak in den Reaktionsraum zugegeben wird, wobei die zugegebene Menge an Ammoniak mindestens 200 mmol und höchstens 3000 mmol pro kg zugegebene Nitroverbindung beträgt und es sich bei der zu hydrierenden Nitroverbindung um Dinitrotoluol handelt.

2. Verfahren nach Anspruch 1, **dadurch gekennzeichnet, dass** die aktive Komponente des Katalysators mindestens ein Element der Gruppe bestehend aus Nickel, Platin, Palladium, Eisen und Kobalt enthält.

3. Verfahren nach Anspruch 1 oder 2, **dadurch gekennzeichnet, dass** die aktive Komponente des Katalysators Chrom enthält.

4. Verfahren nach einem oder mehreren der Ansprüche 1 bis 3, **dadurch gekennzeichnet, dass** die aktive Komponente des Trägerkatalysators Nickel in Form von Nickelkristalliten mit einer bimodalen Nickelkristallitgrößenverteilung enthält und einen Nickel-Gehalt von 60 bis 80 Gew.-% bezogen auf die Gesamtmasse des Katalysators sowie einen Reduktionsgrad von mindestens 70% aufweist.

5. Verfahren nach einem oder mehreren der Ansprüche 1 bis 4, **dadurch gekennzeichnet, dass** die aktive Komponente des Trägerkatalysators ein Gemisch aus Nickel und Platin mit einem Atomverhältnis von Nickel zu Platin von zwischen 30:70 und 70:30 sowie gegebenenfalls eines oder mehrere zusätzliche Metalle enthält.

6. Verfahren nach Anspruch 5, **dadurch gekennzeichnet, dass** der im erfindungsgemäßen Verfahren verwendete Katalysator basierend auf Nickel und Platin und mindestens einem zusätzlichen Metall
1 bis 5 Gew.-% Platin,
0,3 bis 1,5 Gew.-% Nickel,
0,05 bis 1,5 Gew.-% des mindestens einen zusätzlichen Metalls, sowie
94,65 bis 97,45 Gew.-% Trägermaterial
bezogen auf das Gesamtgewicht des Katalysators enthält.

7. Verfahren nach Anspruch 5 oder 6, **dadurch gekennzeichnet, dass** das zusätzliche Metall mindestens ein Metall der Gruppe bestehend aus Kupfer, Kobalt, Eisen, Zink, Mangan und Chrom ist.

8. Verfahren nach Anspruch 7, **dadurch gekennzeichnet, dass** die zugegebene Menge an Ammoniak mindestens 1000 mmol pro kg zugegebene Nitroverbindung beträgt.

9. Verfahren nach einem oder mehreren der Ansprüche 1 bis 8, **dadurch gekennzeichnet, dass** die zugegebene Menge an Ammoniak höchstens 2000 mmol pro kg zugegebene Nitroverbindung beträgt.

10. Verfahren nach einem oder mehreren der Ansprüche 1 bis 9, **dadurch gekennzeichnet, dass** das Ammoniak in flüssiger Form zugegeben wird.

11. Verfahren nach einem oder mehreren der Ansprüche 1 bis 10, **dadurch gekennzeichnet, dass** das Ammoniak als wässrige Lösung zugegeben wird.

12. Verfahren nach einem oder mehreren der Ansprüche 1 oder 11, **dadurch gekennzeichnet, dass** das Reaktionsgemisch neben der zu hydrierenden Nitroverbindung mindestens einen Hochsieder aus der Gruppe bestehend aus Dinitrokresolen, Trinitrokresolen und Nitrophenolen enthält.

13. Verfahren nach einem oder mehreren der Ansprüche 1 bis 11, **dadurch gekennzeichnet, dass** das Reaktionsgemisch neben der zu hydrierenden Nitroverbindung keine Hochsieder aus der Gruppe bestehend aus Dinitrokresolen, Trinitrokresolen und Nitrophenolen enthält.

14. Verfahren nach einem oder mehreren der Ansprüche 1 bis 13, **dadurch gekennzeichnet, dass** das Reaktionsgemisch neben der zu hydrierenden Nitroverbindung mindestens eine Verbindung aus der Gruppe bestehend aus Salpetersäure, Schwefelsäure, Stickstoffoxide, Distickstoffmonoxid, Cyanwasserstoffsäure, Kohlenstoffmonoxid und Nitrobenzoesäure oder deren Abbauprodukte enthält.

15. Verfahren nach einem oder mehreren der Ansprüche 1 bis 14, **dadurch gekennzeichnet, dass** die Hydrierung bei einer Temperatur von 80 bis 250° C durchgeführt wird.

16. Verfahren nach einem oder mehreren der Ansprüche 1 bis 15, **dadurch gekennzeichnet, dass** die Hydrierung in Abwesenheit von Lösungsmitteln durchgeführt wird.

## Claims

1. A process for continuous hydrogenation of a nitroaromatic to the corresponding amine in a liquid reaction mixture comprising the nitro compound in a reaction space in the presence of a supported catalyst which comprises as the active component at least one element from groups 7 to 12 of the periodic table of the elements, wherein ammonia is added to the reaction space during the hydrogenation, wherein the added amount of ammonia is at least 200 mmol and at most 3000 mmol per kg of added nitro compound and the nitro compound for hydrogenation is dinitrotoluene.

2. The process according to claim 1, wherein the active component of the catalyst comprises at least one element from the group consisting of nickel, platinum, palladium, iron and cobalt.

3. The process according to claim 1 or 2, wherein the active component of the catalyst comprises chromium.

4. The process according to one or more of claims 1 to 3, wherein the active component of the supported catalyst comprises nickel in the form of nickel crystallites having a bimodal nickel crystallite size distribution and has a nickel content of 60 to 80 wt% based on the total mass of the catalyst and a degree of reduction of at least 70%.

5. The process according to one or more of claims 1 to 4, wherein the active component of the supported catalyst comprises a mixture of nickel and platinum in an atomic ratio of nickel to platinum of between 30:70 and 70:30 and optionally one or more additional metals.

6. The process according to claim 5, wherein the catalyst based on nickel and platinum and at least one additional metal and used in the process according to the invention comprises
1 to 5 wt% of platinum,
0.3 to 1.5 wt% of nickel,
0.05 to 1.5 wt% of the at least one additional metal and
94.65 to 97.45 wt% of support material
based on the total weight of the catalyst.

7. The process according to claim 5 or 6, wherein the additional metal is at least one metal from the group consisting of copper, cobalt, iron, zinc, manganese and chromium.

8. The process according to claim 7, wherein the added amount of ammonia is at least 1000 mmol per kg of added nitro compound.

9. The process according to one or more of claims 1 to 8, wherein the added amount of ammonia is at most 2000 mmol per kg of added nitro compound.

10. The process according to one or more of claims 1 to 9, wherein the ammonia is added in liquid form.

11. The process according to one or more of claims 1 to 10, wherein the ammonia is added as an aqueous solution.

12. The process according to one or more of claims 1 or 11, wherein in addition to the nitro compound for hydrogenation the reaction mixture comprises at least one high boiler from the group consisting of dinitrocresols, trinitrocresols and nitrophenols.

13. The process according to one or more of claims 1 to 11, wherein in addition to the nitro compound for hydrogenation the reaction mixture comprises no high boilers from the group consisting of dinitrocresols, trinitrocresols and nitrophenols.

14. The process according to one or more of claims 1 to 13, wherein in addition to the nitro compound for hydrogenation the reaction mixture comprises at least one compound from the group consisting of nitric acid, sulfuric acid, nitrogen oxides, dinitrogen monoxide, hydrocyanic acid, carbon monoxide and nitrobenzoic acid or decomposition products thereof.

15. The process according to one or more of claims 1 to 14, wherein the hydrogenation is performed at a temperature of 80°C to 250°C.

16. The process according to one or more of claims 1 to 15, wherein the hydrogenation is performed in the absence of solvents.

## Revendications

1. Procédé d'hydrogénation continue d'un composé nitroaromatique en l'amine correspondante dans un mélange réactionnel liquide comprenant le composé nitré dans un espace réactionnel en présence d'un catalyseur supporté comprenant, comme constituant actif, au moins un élément des groupes 7 à 12 du tableau périodique des éléments, **caractérisé en ce qu'**on ajoute de l'ammoniac dans l'espace réactionnel pendant l'hydrogénation, la quantité d'ammoniac ajoutée étant d'au moins 200 mmol et d'au plus 3000 mmol par kg de composé nitré ajouté et le composé nitré à hydrogéner étant le dinitrotoluène.

2. Procédé selon la revendication 1, **caractérisé en ce que** le constituant actif du catalyseur comprend au moins un élément du groupe constitué par le nickel, le platine, le palladium, le fer et le cobalt.

3. Procédé selon la revendication 1 ou 2, **caractérisé en ce que** le constituant actif du catalyseur comprend du chrome.

4. Procédé selon l'une ou plusieurs des revendications 1 à 3, **caractérisé en ce que** le constituant actif du catalyseur supporté contient du nickel sous forme de cristallites de nickel présentant une répartition granulométrique bimodale des cristallites de nickel et une teneur en nickel de 60 à 80 % en poids par rapport à la masse totale du catalyseur et un degré de réduction d'au moins 70 %.

5. Procédé selon l'une ou plusieurs des revendications 1 à 4, **caractérisé en ce que** le constituant actif du catalyseur supporté comprend un mélange de nickel et de platine ayant un rapport atomique nickel/platine compris entre 30:70 et 70:30 et éventuellement d'un ou plusieurs métaux supplémentaires.

6. Procédé selon la revendication 5, **caractérisé en ce que** le catalyseur utilisé dans le procédé selon l'invention est à base de nickel et de platine et d'au moins un métal supplémentaire et comprend
1 à 5 % en poids de platine,
0,3 à 1,5 % en poids de nickel,
0,05 à 1,5 % en poids de l'au moins un métal supplémentaire, et
94,65 à 97,45 % en poids de matériau de support
par rapport au poids total du catalyseur.

7. Procédé selon la revendication 5 ou 6, **caractérisé en ce que** le métal supplémentaire est au moins un métal du groupe constitué par le cuivre, le cobalt, le fer, le zinc, le manganèse et le chrome.

8. Procédé selon la revendication 7, **caractérisé en ce que** la quantité d'ammoniac ajoutée est d'au moins 1000 mmol par kg de composé nitré ajouté.

9. Procédé selon l'une ou plusieurs des revendications 1 à 8, **caractérisé en ce que** la quantité d'ammoniac ajoutée est d'au plus 2000 mmol par kg de composé nitré ajouté.

10. Procédé selon l'une ou plusieurs des revendications 1 à 9, **caractérisé en ce que** l'ammoniac est ajouté sous forme liquide.

11. Procédé selon l'une ou plusieurs des revendications 1 à 10, **caractérisé en ce que** l'ammoniac est ajouté sous forme de solution aqueuse.

12. Procédé selon l'une ou plusieurs des revendications 1 et 11, **caractérisé en ce que** le mélange réactionnel contient, outre le composé nitré à hydrogéner, au moins un composé à haut point d'ébullition choisi dans le groupe constitué par des dinitrocrésols, des trinitrocrésols et des nitrophénols.

13. Procédé selon l'une ou plusieurs des revendications 1 à 11, **caractérisé en ce que** le mélange réactionnel ne contient pas, outre le composé nitré à hydrogéner, de produits à haut point d'ébullition choisis dans le groupe constitué par des dinitrocrésols, des trinitrocrésols et des nitrophénols.

14. Procédé selon l'une ou plusieurs des revendications 1 à 13, **caractérisé en ce que** le mélange réactionnel contient, outre le composé nitré à hydrogéner, au moins un composé choisi dans le groupe constitué par l'acide nitrique, l'acide sulfurique, des oxydes d'azote, le monoxyde de diazote, l'acide cyanhydrique, le monoxyde de carbone et l'acide nitrobenzoïque ou leurs produits de dégradation.

15. Procédé selon l'une ou plusieurs des revendications 1 à 14, **caractérisé en ce que** l'hydrogénation est effectuée à une température de 80 à 250 °C.

16. Procédé selon l'une ou plusieurs des revendications 1 à 15, **caractérisé en ce que** l'hydrogénation est effectuée en l'absence de solvants.
